# FASCICULE DE BREVET EUROPEEN

(11) **EP 0 625 966 B1**
(45) Date de publication et mention de la délivrance du brevet: **27.08.1997**
(21) Numéro de dépôt: 94902821.1
(22) Date de dépôt: 16.12.1993
(51) Int. Cl.: C07C 271/22, A61K 7/48

(54) **NOUVEAUX SELS D'AMINES PRIMAIRES DERIVES D'ACIDES AMINES A GROUPEMENT URETHANNE, ET LEUR UTILISATION DANS DES COMPOSITIONS COSMETIQUES**
SALZE VON URETHANAMINOSÄUREDERIVATEN MIT PRIMÄREN AMINEN UND IHRE VERWENDUNG IN KOSMETISCHEN ZUSAMMENSETZUNGEN
SALTS OF URETHANE AMINOACID DERIVATIVES WITH PRIMARY AMINES AND THEIR USE IN COSMETIC COMPOSITIONS

(30) Priorité: 16.12.1992 FR 9215162
(43) Date de publication de la demande: 30.11.1994
(73) Titulaire: L'OREAL, 75008 Paris (FR)
(72) Inventeur: GREGOIRE, Nathalie, F-92330 Sceaux (FR); CANDAU, Didier, F-77000 Melun (FR); QUEMIN, Eric, F-93420 Villepinte (FR)
(74) Mandataire: Tonnellier, Jean-Claude
(86) Numéro de dépôt international: FR9301255
(87) Numéro de publication internationale: WO9413628

(56) Documents cités:
- EP-A- 0 408 448
- EP-A- 0 468 856
- FR-A- 2 604 625

## Description

L'invention a pour objet de nouveaux sels d'amines primaires d'uréthannes dérivés d'acides aminés, et leur utilisation notamment comme agents émulsionnants et hydratants dans des compositions cosmétiques.

Dans la demande de brevet FR 89 09328 (n° de publication 2 649 697) on a décrit des uréthannes dérivés de certains acides aminés ou des sels desdits acides, comme agents hydratants ou tensioactifs dans des compositions cosmétiques ou pharmaceutiques destinées au traitement des peaux sèches. Dans cette demande de brevet antérieure, on décrit la préparation de certains sels, notamment des sels métalliques, des sels d'ammonium et des sels d'amines tertiaires.

Cependant l'utilisation des dérivés d'uréthanne et de leurs sels décrits dans la demande de brevet antérieure pose des problèmes particuliers de formulation. Ces produits, sous forme acide, sont très peu solubles dans l'eau à température ambiante. Par chauffage, par exemple à 80°C environ, on arrive à former un gel sensiblement homogène. Mais le pH acide d'un tel gel ne permet pas de formuler ces produits dans des compositions pour la peau. En outre, quand on refroidit à la température ambiante, l'acide reprécipite.

Lorsqu'on essaie de contourner cette difficulté en salifiant à l'aide d'une base comme l'hydroxyde de sodium ou la triéthanolamine, il faut chauffer la phase aqueuse contenant l'acide. Quand le gel est formé, on ajoute goutte à goutte une solution aqueuse de la base choisie et, lorsque la stoechiométrie est atteinte, on obtient une solution limpide du sel formé. Toutefois, lors du refroidissement à température ambiante, on observe la formation d'un précipité lors du refroidissement ou au bout de quelques jours au plus.

On a alors cherché à solubiliser lesdits dérivés d'uréthanne dans un mélange d'eau et d'huile, en formant une émulsion à chaud.

Avec les dérivés non salifiés, l'observation au microscope montre qu'on obtient une émulsion très fine à 80°C. Toutefois, par refroidissement à température ambiante, la texture devient complètement hétérogène, avec notamment une précipitation de cristaux.

Les sels desdits dérivés d'uréthanne permettent également d'obtenir des émulsions fines. Toutefois, avec les sels d'amines tertiaires, tels que le sel de triéthanolamine, l'émulsion se sépare en deux phases au bout de quinze jours environ.

On a par contre découvert que, de façon surprenante, avec certains desdits dérivés d'uréthanne de la sérine ou de la thréonine, salifiés avec une amine primaire, il est possible d'obtenir des émulsions restant stables pendant plusieurs mois.

L'invention a donc pour objet les sels d'amines primaires des dérivés de formule (I):

R'O-CO-NH-CH(R)-COOH (I)

dans laquelle :
R représente -CH₂OH ou -CHOH-CH₃,
et R' représente un groupement alkyle, éventuellement insaturé, ayant au moins 10 atomes de carbone ainsi que les produits de salification au moins partielle des dérivés de formule (I) avec des amines primaires, et dont le degré de salification est suffisant pour obtenir, en solution aqueuse, un pH supérieur à 6 et inférieur à 7,5.

Par produits de salification on entend ici les composés obtenus par salification des composés de formule (I) avec une amine primaire dans des proportions qui ne sont pas nécessairement stoechiométriques. Toutefois, dans la présente demande, l'expression "sels d'amine primaire" désignera, sauf mention contraire, aussi bien les sels proprements dits que les autres produits de salification, et vice versa.

Dans des modes de réalisation particuliers, les sels d'amines primaires des dérivés de formule I utilisables selon l'invention peuvent présenter les caractéristiques suivantes, prises isolément ou, le cas échéant, en combinaison :
- R' comporte de 10 à 20 atomes de carbone, et en particulier de 14 à 18 atomes de carbone et notamment 16 atomes de carbone ;
- lesdites amines primaires répondent à la formule (II) :

   H₂N-CR₁(R₂)(R₃) (II)

   dans laquelle :
   R₁ représente -CH₂OH, et R₂ et R₃ représentent indépendamment un alkyle inférieur éventuellement hydroxylé ; R₂ représente par exemple -CH₃ ou -CH₂OH, et R₃ peut représenter -CH₃, -C₂H₅ ou -CH₂OH ;
- l'amine primaire (II) est choisie parmi le 2-amino 2-méthyl propane-1,3-diol, le 2-amino 2-méthyl propane-1-ol et le 2-amino 2-hydroxyméthyl propane-1,3-diol ;
- ou bien l'amine primaire utilisée pour la salification des dérivés de formule I peut elle-même être un acide aminé contenant au moins deux groupements aminés, comme par exemple la lysine ou l'arginine.

Le procédé de préparation des composés de formule I et de leurs sels est décrit dans la demande de brevet français 89 09328 (numéro de publication 2 649 697). On rappelle que ce procédé est caractérisé par l'on fait que l'on fait réagir un sel d'un acide aminé (D-, L- ou DL) choisi parmi la sérine et la thréonine, avec un composé de formule (III) :

X-CO-OR' (III)

X représentant un halogène ou un groupement 1-imidazolyle, dans un solvant approprié, que l'on isole selon les méthodes connues le dérivé d'uréthanne correspondant de formule I formé et que l'on transforme selon les méthodes connues ledit dérivé d'uréthanne en sel correspondant.

Le sel de départ est par exemple un sel de métal alcalin ou un sel d'amine telle que la triéthylamine.

La réaction entre le sel d'acide aminé et l'halogénoformiate (de préférence chloroformiate) peut être effectuée à température ambiante dans un solvant classique tel qu'un mélange eau-tétrahydrofuranne, eau-dioxanne ou eau-pyridine.

La réaction entre le sel d'acide aminé et le dérivé d'imidazole de formule III peut être effectuée par exemple dans le N,N-diméthylformamide ou le N,N-diméthylacétamide à une température de 20 à 100°C, par exemple à 60°C, en présence d'un catalyseur basique tel que le t-butanolate de potassium ou l'imidazolidure de sodium.

Les sels d'amines primaires ou produits de salification des composés de formule I ont des propriétés cosmétiques intéressantes, notamment des propriétés d'hydratation de la peau. Ils ont également des propriétés tensioactives qui permettent de les utiliser comme agents émulsionnants. Ils ont en outre des propriétés bactéricides.

Les sels ou produits de salification de l'invention peuvent être utilisés comme agents d'hydratation de la peau chez les humains. Ils permettent de conserver ou de restaurer la souplesse de la peau, son élasticité, sa résistance aux mouvements du corps et sa fonction de barrière à l'entrée de substances toxiques. On sait que les compositions cosmétiques ou dermopharmaceutiques destinées à hydrater la peau sont utilisées notamment chez les personnes ayant une peau dite sèche. Ce phénomène est caractérisé par une peau ayant un taux d'évaporation nettement plus élevé que celui d'une peau saine, par une perte de l'élasticité cutanée et par la formation de rides. Il peut être provoqué notamment par des troubles pathologiques de la kératinisation, par le vieillissement ou par l'exposition excessive au soleil ou à divers agents extérieurs (détergents, savons, solvants, atmosphère sèche, etc.). Ce phénomène peut affecter toutes les parties du corps et particulièrement le visage, le cou et les mains.

Les propriétés des sels de l'invention, notamment lorsqu'ils sont appliqués dans des compositions sous forme d'émulsions, peuvent être mises en évidence par différents essais. C'est ainsi que l'influence favorable sur l'élasticité de la peau peut être montrée en utilisant l'appareillage décrit dans l'article de L. Rasseneur et al., Int. J. of Cosm. Sci. 4, 247-260, (1982).

Les propriétés des produits de salification des dérivés de formule I ont été également mises en évidence à l'aide d'un évaporimètre (Servomed) qui est un appareil permettant la détermination quantitative de l'évaporation d'eau à partir d'un échantillon de stratum corneum obturant une capsule cylindrique contenant de l'eau, le tout étant placé dans une chambre à température et humidité relative contrôlées. Des capteurs permettent de mesurer la pression partielle de la vapeur d'eau en deux points situés à des distances différentes de l'échantillon. On peut déterminer ainsi le gradient de pression partielle de vapeur d'eau entre les deux points, et donc le taux d'évaporation, conformément à la loi de Fick.

Ces études ont montré que les agents de l'invention réduisent le taux d'évaporation de la peau.

On a également pu mettre en évidence l'augmentation, grâce aux agents hydratants de l'invention, du taux d'hydratation de la peau par mesure de sa conductivité. On effectue cette mesure avec un appareil comportant une électrode centrale sous forme de tige entourée par une électrode cylindrique. L'appareil est appliqué sur la peau. On applique un courant alternatif de haute fréquence. On constate que plus l'hydratation de la peau est importante, plus la quantité de courant consommé est élevée. On peut ainsi mettre en évidence l'augmentation de la conductivité de la peau et donc l'augmentation de son taux d'hydratation grâce aux compositions de l'invention.

Il convient de noter enfin que dans un test non public réalisé sur 60 utilisatrices à peau sèche, au bout d'un mois d'application quotidienne d'une émulsion contenant au moins un produit de salification d'un composé de formule I avec une amine primaire, 80% de ces utilisatrices ont perçu une activité hydratante.

Ces diverses propriétés des sels de l'invention permettent de les utiliser avantageusement dans des compositions cosmétiques ou dermopharmaceutiques, sous forme d'émulsions, destinées notamment à améliorer l'aspect des peaux sèches.

Les compositions sous forme d'émulsions contenant lesdits sels d'amines primaires sont stables, contrairement aux émulsions obtenues avec les dérivés d'uréthanne décrits jusqu'à présent.

L'invention a donc également pour objet une composition cosmétique ou dermopharmaceutique qui se présente sous la forme d'une émulsion et qui contient au moins un sel d'amine primaire ou produit de salification avec une amine primaire, tel que défini précédemment.

La composition de l'invention contient par exemple, de 1 à 10 %, et en particulier de 2 à 5 %, en poids, d'au moins un desdits sels ou produits de salification.

Les compositions de l'invention contiennent généralement, en poids, au moins 10% d'huile, et le plus souvent de 20 à 80%.

Toutes les huiles utilisées dans la réalisation de compositions cosmétiques conviennent à la réalisation sous forme d'émulsions de l'invention. On peut citer par exemple les huiles minérales telles que l'huile de vaseline ; les huiles végétales, par exemple les huiles de noyaux d'abricot, l'huile de jojoba ; les esters gras ; les huiles siliconées, y compris les huiles de silicones volatiles.

En raison des propriétés émulsionnantes des sels d'amines primaires des dérivés de formule I, il est possible de réaliser des compositions exemptes d'autres agents émulsionnants.

Il est toutefois possible d'utiliser, pour la préparation des compositions sous forme d'émulsions de l'invention, un agent co-émulsionnant. De préférence les sels d'amines primaires ou produits de salification sont présents dans la composition à raison d'au moins 10% en moles, et de préférence à raison d'au moins 50% en moles par rapport à l'ensemble des émulsionnants (c'est-à-dire sels d'amines primaires + co-émulsionnants).

Parmi les agents co-émulsionnants, on peut citer par exemple les dérivés d'acides ou alcools gras insaturés ou ramifiés, le cholestérol et l'α-phytanetriol. On peut utiliser notamment l'acide oléique, linoléique, linolénique ou isostéarique, l'alcool oléique, l'alcool isostéarylique, et les oléates et isostéarates mono- ou poly-glycérolés.

Pour préparer la composition sous forme d'émulsion, de l'invention on peut par exemple chauffer la phase aqueuse contenant le dérivé de formule I à une température suffisante, par exemple 80°C, pour faciliter sa solubilisation. L'amine primaire utilisée pour la salification peut être présente dans le produit de départ ou ajoutée après chauffage. L'amine primaire est ajoutée en quantité suffisante pour que le pH du produit final soit dans la gamme de 6 à 7,5. Bien entendu, si la composition contient d'autres produits acides (par exemple des agents épaississants de type Carbopols) la quantité d'amine primaire doit être suffisante pour neutraliser à la fois les dérivés de formule I et les autres produits acides présents dans la composition. On ajoute ensuite, sous agitation forte, la phase huileuse pour créer une émulsion.

Si on utilise des agents co-émulsionnants, ceux-ci sont ajoutés de préférence avec la phase huileuse.

Les compositions de l'invention peuvent également renfermer des agents épaississants ou gélifiants pour la phase aqueuse, par exemple à raison de 0,5 à 10% en poids par rapport au poids total de la composition. Les agents épaississants peuvent être constitués par des dérivés de cellulose, des polymères acryliques, des alginates, des gommes telles que la gomme de xanthane, de guar, de caroube, la gomme arabique, ou bien des polyéthylèneglycols.

Les compositions de l'invention peuvent en outre contenir d'autres agents hydratants ou humectants connus, tels que la glycérine, la triacétine, ou plus généralement d'autres ingrédients actifs tels que des agents actifs contre le vieillissement de la peau.

Les compositions de l'invention peuvent également contenir des adjuvants usuels tels que des agents anti-oxydants, des parfums, des colorants, etc...

Parmi les anti-oxydants utilisables, on citera la tert-butylhydroquinone, le butylhydroxytoluène et l'alpha-tocophérol et ses dérivés.

Les sels d'amines primaires des dérivés de formule I ont également des propriétés bactéricides qui permettent de réaliser des compositions sans addition d'autres agents conservateurs. On peut toutefois utiliser bien entendu les agents conservateurs usuels tels que les p-hydroxybenzoates de méthyle, d'éthyle, de propyle, de butyle et d'isobutyle (parabens), le 2-phénoxy éthanol, l'acide sorbique, le sorbate de potassium,le diiséthionate d'hexamidine, l'imidazolidinyl urée (nom commercial Germall 115), ou encore les agents conservateurs commercialisés sous les dénominations Kathon et Triclosan.

Lorsque les sels d'amines primaires ou produits de salification des dérivés de formule I sont utilisés comme agents émulsionnants majoritaires, les émulsions obtenues sont des émulsions du type huile-dans-l'eau. Toutefois, avec des agents co-émulsionnants ayant une valeur de HLB (balance hydrophile-lipophile) convenable, il est possible d'obtenir également des compositions sous la forme eau-dans-l'huile.

Les compositions de l'invention peuvent être des émulsions de consistance liquide ou semi-liquide du type laits obtenues par dispersion d'une phase grasse dans une phase aqueuse ou inversement ; ou des suspensions ou émulsions de consistance molle du type crèmes.

Les compositions de l'invention sont notamment des émulsions blanches constituant des crèmes de soin hydratantes, des laits corporels, des démaquillants pour le visage, des crèmes contenant divers agents actifs, par exemple des crèmes avec agents actifs amincissants (notamment caféine) ou agents anti-acnéiques (par exemple thioxolone), ou agents kératolytiques (par exemple l'acide salicylique). Lorsqu'elles sont exemptes d'huiles autres que les huiles de silicones, ces compositions peuvent également constituer des crèmes pour peaux grasses.

Les compositions de l'invention peuvent constituer également des émulsions teintées usuelles telles que des fonds de teint, des compositions fluides hydratantes pour le soin de la peau, des ligneurs (ou eye-liners), etc.

L'invention a également pour objet l'utilisation d'au moins un sel ou produit de salification tel que défini précédemment comme agent émulsionnant ou co-émulsionnant permettant d'améliorer la stabilité des émulsions devant contenir des dérivés de formule I. Bien entendu, il s'agit ici d'émulsions dans lesquelles les dérivés de formule I (sous forme de sels d'amines primaires), tout en jouant le rôle d'agents émulsionnants, sont utilisés en raison de leur propriété d'hydratation de la peau.

Les exemples suivants illustrent l'invention.

Dans ces exemples, le composé A représente

CH₃-(CH₂)₁₅-O-CO-NH-CH(CH₂OH)-COOH (A)

Le composé B représente le 2-amino 2-hydroxyméthyl propane-1,3-diol.

### EXEMPLE 1 : Crème de soin

- Composé de formule A 5 g
- Composé B 1,66 g
- Parahydroxybenzoate de méthyle 0,2 g
- Huile de noyaux d'abricot 38,8 g
- Parahydroxybenzoate de propyle 0,2 g
- Cyclométhicone D5 30 g
- Eau qsp 100 g

Cette crème de soin est destinée au traitement des peaux sèches.

Pour préparer cette crème, on opère de la façon suivante : les composés A et B sont mélangés à la phase aqueuse à une température de 80°C jusqu'à complète dissolution. On verse la phase huileuse sur la phase aqueuse sous agitation vigoureuse, toujours à une température de 80°C, afin d'obtenir une émulsion.

De façon analogue, on a préparé les compositions des exemples suivants.

### EXEMPLE 2 : crème de soin

- Composé A 2,5 g
- Composé B 1,44 g
- Huile de noyaux d'abricot 19,8 g
- Huile de jojoba 5 g
- Cyclométhicone D5 5 g
- Gomme de silicone vendue sous la dénomination Q₂1401 par la société Dow Corning 5 g
- Parahydroxybenzoate de propyle 0,2 g
- Carbopol 980 (Goodrich) 0,75 g
- Parahydroxybenzoate de méthyle 0,2 g
- Eau qsp 100 g

Cette crème de soin à la fois légère et hydratante est particulièrement adaptée aux peaux sèches et déshydratées.

### EXEMPLE 3 : lait corporel

- Composé A 2,5 g
- Aminométhylpropanol (AMP) 1,3 g
- Acide isostéarique 1,94 g
- Huile de jojoba 20 g
- cyclométhicone D5 15 g
- Carbopol 980 0,5 g
- Gomme de xanthane 0,2 g
- Parahydroxybenzoate de méthyle 0,2 g
- Parahydroxybenzoate de propyle 0,2 g
- Eau qsp 100 g

Ce lait corporel de structure très légère convient aux peaux normales et sèches qui retrouvent rapidement un taux d'hydratation élevé.

### EXEMPLE 4 : fond de teint

- Composé A 2,5 g
- Composé B 1,44 g
- Carbopol 980 0,75 g
- Parahydroxybenzoate de méthyle 0,2 g
- Huile de noyaux d'abricot 25 g
- Cyclométhicone D5 9,8 g
- Parahydroxybenzoate de propyle 0,2 g
- Oxyde de fer noir 0,11 g
- Oxyde de fer rouge 0,39 g
- Oxyde de fer jaune 0,6 g
- Dioxyde de titane 2,9 g
- Eau qsp 100 g

Ce fond de teint de texture très légère a un effet hydratant, comme une crème de soin. Il convient particulièrement aux peaux sèches et déshydratées.

### EXEMPLE 5 : Crème pour peau à tendance grasse

- Composé A 2,5 g
- AMP 1,5 g
- Glycérine 3 g
- Acide oléique 1,94 g
- Cyclométhicone D5 35 g
- Carbopol 980 0,75 g
- Parahydroxybenzoate de méthyle 0,2 g
- Parahydroxybenzoate de propyle 0,1 g
- Eau qsp 100 g

### EXEMPLE 6 : Crème fluide

- Composé A 1,8 g
- A.M.P. 1,26 g
- Huile de noyaux d'abricot 15 g
- Huile de silicone volatile (Cyclométhicone D5) 10 g
- Acide isostéarique 1,2 g
- Carbopol 980 0,75 g
- Conservateurs 0,3 g
- Eau qsp 100 g

Cette crème est souple et très légère à l'application. Elle convient en particulier aux peaux normales et grasses.

### EXEMPLE 7 : Crème de soin adoucissante

- Composé A 2,5 g
- Lysine 2,73 g
- Acide oléique 1,94 g
- Huile de noyaux d'abricot 19,7 g
- Cyclométhicone D5 15 g
- Propylène glycol 5 g
- Conservateurs 0,3 g
- Eau qsp 100 g

Cette crème, particulièrement douce, convient aux peaux sèches et sensibles, qu'elle hydrate.

### EXEMPLE 8 : Crème pour peaux sèches

- N-dodécyloxycarbonylsérine 5 g
- Composé B 1,98 g
- Conservateurs (parabens) 0,3 g
- Huile de noyaux d'abricot 70 g
- Eau qsp 100 g

Cette crème , à effet hydratant, est destinée aux peaux sèches.

## Revendications

1. Les sels d'amines primaires des dérivés de formule (I) :
R'O-CO-NH-CH(R)-COOH (I)
dans laquelle :
R représente -CH₂OH ou -CHOH-CH₃,
et R' représente un groupement alkyle, éventuellement insaturé, ayant au moins 10 atomes de carbone, ainsi que les produits de salification au moins partielle des dérivés de formule (I) avec des amines primaires, et dont le degré de salification est suffisant pour obtenir, en solution aqueuse, un pH supérieur à 6 et inférieur à 7,5.

2. Sels ou produits de salification selon la revendication 1, caractérisés par le fait que R' comporte de 10 à 20 atomes de carbone, et en particulier de 14 à 18 atomes de carbone.

3. Sels ou produits de salification selon l'une quelconque des revendications précédentes, caractérisés par le fait que R' comporte 16 atomes de carbone.

4. Sels ou produits de salification selon l'une quelconque des revendications precédentes, caractérisés par le fait que lesdites amines primaires répondent à la formule (II) :
H₂N-CR₁(R₂)(R₃) (II)
dans laquelle :
R₁ représente -CH₂OH, et R₂ et R₃ représentent indépendamment un alkyle inférieur éventuellement hydroxylé.

5. Sels ou produits de salification selon la revendication précédente, caractérisés par le fait que R₂ représente -CH₃ ou -CH₂OH, et R₃ représente -CH₃, -C₂H₅ ou -CH₂OH.

6. Sels ou produits de salification selon l'une quelconque des revendications précédentes, caractérisés par le fait que l'amine primaire est choisie parmi le 2-amino 2-méthyl propane-1,3-diol, le 2-amino 2-méthyl propane-1-ol et le 2-amino 2-hydroxyméthyl propane-1,3-diol.

7. Sels ou produits de salification selon l'une quelconque des revendications 1 à 3, caractérisés par le fait que ladite amine primaire est un acide aminé contenant au moins deux groupements aminés.

8. Sels ou produits de salification selon la revendication précédente, caractérisés par le fait que ladite amine primaire est choisie parmi la lysine et l'arginine.

9. Composition cosmétique ou dermopharmaceutique caractérisée par le fait qu'elle se présente sous la forme d'une émulsion et qu'elle contient au moins un sel ou produit de salification tel que défini dans l'une quelconque des revendications précédentes.

10. Composition selon la revendication précédente caractérisée par le fait qu'elle contient de 1 à 10 %, et en particulier de 2 à 5 %, en poids, d'au moins un desdits sels ou produits de salification.

11. Composition selon la revendication 9 ou 10, caractérisée par le fait qu'elle est exempte d'agent émulsionnant autre que ledit sel ou ledit produit de salification.

12. Composition selon l'une quelconque des revendications 9 et 10, caractérisée par le fait qu'elle contient en outre un agent co-émulsionnant.

13. Composition selon la revendication 12, caractérisée par le fait que ledit sel ou produit de salification est présent à raison d'au moins 10% en moles et en particulier d'au moins 50% en moles par rapport à l'ensemble des émulsionnants.

14. Composition selon l'une quelconque des revendications 12 et 13, caractérisée par le fait que ledit agent co-émulsionnant est choisi parmi les dérivés d'acides ou alcools gras insaturés ou ramifiés, le cholestérol et l'α-phytanetriol.

15. Composition selon l'une quelconque des revendications 9 à 14, caractérisée par le fait qu'elle contient en poids au moins 10% d'huile et en particulier de 20 à 80%.

16. Utilisation d'au moins un sel ou produit de salification tel que défini dans l'une quelconque des revendications 1 à 8, comme agent émulsionnant ou co-émulsionnant permettant d'améliorer la stabilité des compositions cosmétiques sous forme d'émulsions.

17. Utilisation selon la revendication précédente, caractérisée par le fait que lesdits sels ou produits de salification sont utilisés comme seuls agents émulsionnants.

18. Utilisation selon la revendication 16, caractérisée par le fait que l'on utilise ledit sel ou le produit de salification avec au moins un co-émulsionnant.

19. Utilisation selon la revendication précédente, caractérisée par le fait ledit co-émulsionnant est tel que défini dans la revendication 14.

20. Utilisation selon l'une quelconque des revendications 16 à 19, caractérisée par la fait que ledit sel ou produit de salification est présent à raison d'au moins 10%, et en particulier d'au moins 50% en moles par rapport à l'ensemble des émulsionnants.

## Patentansprüche

1. Salze von primären Aminderivaten gemäß der Formel (I):
R'O-CO-NH-CH(R)-COOH (I)
worin
R den Rest -CH₂OH oder -CHOH-CH₃ bedeutet, wobei
R' einen gegebenenfalls ungesättigten Alkylrest mit mindestens 10 Kohlenstoffatomen wie auch Produkte einer mindestens teilweisen Versalzung von Derivaten gemäß der Formel (I) mit primären Aminen (Aminosäuren) darstellt, wobei der Versalzungsgrad ausreicht, um in wäßriger Lösung einen pH-Wert von höher als 6 und niedriger als 7,5 zu erhalten.

2. Salze oder Versalzungsprodukte nach Anspruch 1, dadurch gekennzeichnet, daß R' 10 bis 20 Kohlenstoffatome, insbesondere 14 bis 18 Kohlenstoffatome aufweist.

3. Salze oder Versalzungsprodukte nach einem der vorstehenden Ansprüche, dadurch gekennzeichnet, daß R' 16 Kohlenstoffatome aufweist.

4. Salze oder Versalzungsprodukte nach einem der vorstehenden Ansprüche, dadurch gekennzeichnet, daß die primären Amine der Formel (II) entsprechen:
H₂N-CR₁(R₂)(R₃) (II)
worin
R₁ die Gruppe -CH₂OH bedeutet, wobei R₂ und R₃ unabhängig voneinander einen gegebenenfalls hydroxylierten niederen Alkylrest darstellen.

5. Salze oder Versalzungsprodukte nach dem vorstehenden Anspruch, dadurch gekennzeichnet, daß R₂ die Gruppe -CH₃ oder -CH₂OH, und R₃ die Gruppe -CH₃,-C₂H₅ oder -CH₂OH bedeuten.

6. Salze oder Versalzungsprodukte nach einem der vorstehenden Ansprüche, dadurch gekennzeichnet, daß das primäre Amin unter 2-Amino-2-methylpropan-1,3-diol, 2-Amino-2-methylpropan-1-ol und 2-Amino-2-hydroxymethylpropan-1,3-diol ausgewählt ist.

7. Salze oder Versalzungsprodukte nach einem der Ansprüche 1 bis 3, dadurch gekennzeichnet, daß das primäre Amin eine Aminosäure mit einem Gehalt an mindestens zwei Aminogruppen darstellt.

8. Salze oder Versalzungsprodukte nach dem vorstehenden Anspruch, dadurch gekennzeichnet, daß das primäre Amin unter Lysin und Arginin ausgewählt ist.

9. Kosmetische oder dermatopharmazeutische Zubereitung, dadurch gekennzeichnet, daß sie in Form einer Emulsion vorliegt, wobei diese mindestens ein Salz oder Versalzungsprodukt enthält, deren Bedeutung in einem der vorstehenden Ansprüche angegeben ist.

10. Zubereitung nach dem vorstehenden Anspruch, dadurch gekennzeichnet, daß sie 1 bis 10 Gew.-%, insbesondere 2 bis 5 Gew.-%, mindestens eines der genannten Salze oder Versalzungsprodukte enthält.

11. Zubereitung nach Anspruch 9 oder 10, dadurch gekennzeichnet, daß sie außer dem genannten Salz oder Versalzungsprodukt keinen anderen Emulgator enthält.

12. Zubereitung nach einem der Ansprüche 9 und 10, dadurch gekennzeichnet, daß sie zusätzlich noch einen Co-Emulgator enthält.

13. Zubereitung nach Anspruch 12, dadurch gekennzeichnet, daß das genannte Salz oder Versalzungsprodukt in einem Mengenverhältnis von mindestens 10 Mol-%, insbesondere in einem Mengenverhältnis von mindestens 50 Mol-% in bezug auf die Gesamtmenge an Emulgatoren vorhanden ist.

14. Zubereitung nach einem der Ansprüche 12 und 13, dadurch gekennzeichnet, daß der Co-Emulgator aus ungesättigten oder verzweigten Fettsäurederivaten oder Fettalkoholderivaten, dem Cholesterin oder α-Phytantriol ausgewählt ist.

15. Zubereitung nach einem der Ansprüche 9 bis 14, dadurch gekennzeichnet, daß sie mindestens 10 Gew.-% an Öl, insbesondere 20 bis 80 Gew.-% Öl enthält.

16. Verwendung mindestens eines Salzes oder Versalzungsprodukts gemäß Definition in einem der Ansprüche 1 bis 8 als Emulgator oder Co-Emulgator, der die Verbesserung der Stabilität der kosmetischen Zubereitungen in Form von Emulsionen ermöglicht.

17. Verwendung nach dem vorstehenden Anspruch, dadurch gekennzeichnet, daß die genannten Salze oder Versalzungsprodukte als alleinige Emulgatoren eingesetzt werden.

18. Verwendung nach Anspruch 16, dadurch gekennzeichnet, daß das genannte Salz oder Versalzungsprodukt zusammen mit mindestens einem Co-Emulgator verwendet wird.

19. Verwendung nach dem vorstehenden Anspruch, dadurch gekennzeichnet, daß der Co-Emulgator die in Anspruch 14 angegebene Bedeutung aufweist.

20. Verwendung nach einem der Ansprüche 16 bis 19, dadurch gekennzeichnet, daß das genannte Salz oder Versalzungsprodukt in einem Mengenverhältnis von mindestens 10 Mol-%, insbesondere in einem Mengenverhältnis von mindestens 50 Mol-% in bezug auf die Gesamtmenge an Emulgatoren vorhanden ist.

## Claims

1. The primary amine salts of the derivatives of formula (I) :
R'O-CO-NH-CH(R)-COOH (I)
in which:
R represents -CH₂OH or -CHOH-CH₃,
and R' represents an optionally unsaturated alkyl group having at least 10 carbon atoms, as well as the at least partial salification products of the derivatives of formula (I) with primary amines, and the degree of salification of which is sufficient to obtain a pH above 6 and below 7.5 in aqueous solution.

2. Salts or salification products according to Claim 1, characterized in that R' contains from 10 to 20 carbon atoms, and especially from 14 to 18 carbon atoms.

3. Salts or salification products according to either of the preceding claims, characterized in that R' contains 16 carbon atoms.

4. Salts or salification products according to any one of the preceding claims, characterized in that the said primary amines correspond to the formula (II):
H₂N-CR₁(R₂) (R₃) (II)
in which:
R₁ represents -CH₂OH, and R₂ and R₃ independently represent an optionally hydroxylated lower alkyl.

5. Salts or salification products according to the preceding claim, characterized in that R₂ represents -CH₃ or -CH₂OH, and R₃ represents -CH₃, -C₂H₅ or -CH₂OH.

6. Salts or salification products according to any one of the preceding claims, characterized in that the primary amine is chosen from 2-amino-2-methyl-1,3-propanediol, 2-amino-2-methyl-1-propanol and 2-amino-2-hydroxymethyl-1,3-propanediol.

7. Salts or salification products according to any one of Claims 1 to 3, characterized in that the said primary amine is an amino acid containing at least two amino groups.

8. Salts or salification products according to the preceding claim, characterized in that the said primary amine is chosen from lysine and arginine.

9. Cosmetic or dermopharmaceutical composition, characterized in that it takes the form of an emulsion, and in that it contains at least one salt or salification product as defined in any one of the preceding claims.

10. Composition according to the preceding claim, characterized in that it contains from 1 to 10%, and especially from 2 to 5%, by weight of at least one of the said salts or salification products.

11. Composition according to Claim 9 or 10, characterized in that it is free from emulsifying agent other than the said salt or said salification product.

12. Composition according to either of Claims 9 and 10, characterized in that it contains, in addition, a co-emulsifying agent.

13. Composition according to Claim 12, characterized in that the said salt or salification product is present in the proportion of at least 10 mol%, and especially of at least 50 mol%, relative to the collective emulsifiers.

14. Composition according to either of Claims 12 and 13, characterized in that the said co-emulsifying agent is chosen from derivatives of unsaturated or branched fatty acids or alcohols, cholesterol and α-phytanetriol.

15. Composition according to any one of Claims 9 to 14, characterized in that it contains, by weight, at least 10% of oil, and especially from 20 to 80%.

16. Use of at least one salt or salification product as defined in any one of Claims 1 to 8, as emulsifying or co-emulsifying agent enabling the stability of cosmetic compositions in emulsion form to be improved.

17. Use according to the preceding claim, characterized in that the said salts or salification products are used as sole emulsifying agents.

18. Use according to Claim 16, characterized in that the said salt or the salification product is used with at least one co-emulsifier.

19. Use according to the preceding claim, characterized in that the said co-emulsifier is as defined in Claim 14.

20. Use according to any one of Claims 16 to 19, characterized in that the said salt or salification product is present in the proportion of at least 10 mol%, and especially of at least 50 mol%, relative to the collective emulsifiers.
